# EUROPEAN PATENT SPECIFICATION

(11) **EP 3 379 543 B1**
(45) Date of publication and mention of the grant of the patent: **05.11.2025**
(21) Application number: 17206227.5
(22) Date of filing: 08.12.2017
(51) Int. Cl.: G16H 50/20, G16H 10/20, G16H 10/60, G16H 20/10, G16H 40/20, G16H 50/30, G16H 50/50, G16H 70/60

(54) **APPARATUS, METHOD AND COMPUTER PROGRAM FOR PROVIDING MEDICAL ADVICE BASED ON SELF-REPORTED SYMPTOMS OF A USER**
VORRICHTUNG, VERFAHREN UND COMPUTERPROGRAMM
APPAREIL, PROCÉDÉ ET PROGRAMME INFORMATIQUE

(30) Priority: 24.03.2017 GB 201704727
(43) Date of publication of application: 26.09.2018
(73) Proprietor: Clinova Limited, Southampton SO18 2RZ (GB)
(72) Inventor: KARIM, Arsalan, Shipley, BD18 4AR (GB); EBUBEDIKE, Charles, London, SW16 2TT (GB); NAEEM, Adil, Bristol, BS16 2LH (GB)
(74) Representative: Page White Farrer

(56) References cited:
- US-A1- 2015 149 202
- US-B1- 6 396 931

## Description

### Field of the invention

The present application relates to an apparatus and computer program. More particularly the present application relates to an apparatus, method and computer program for providing medical advice, based upon one or more medical symptoms of a user.

### Background

Today, the primary starting point for medical care is arranging a one-on-one GP (general practitioner) or physician appointment, or turning up in the emergency room of a hospital. Both of these options are time, labour and cost-intensive for both the patient and the health service. Amid squeezed budgets and rising demand, many patients cannot see a doctor when they need to. Typically, one in eight patients is misdiagnosed. Medical error, which kills about 1,000 people a month in the UK, is also the third leading cause of death in the US, according to a 2016 study in the British Medical Journal. In 2016, 53 million people in the UK went to see a GP who could have been suitable for self-care treatment, and 2.3 million people went to an accident and emergency (A&E) department who could have been suitable for self-care treatment.

US2015/0149202A1 discloses a method of providing medical advice via the Internet. Accurate data collection and a summary of medical information is constructed by using a combination of Virtual Resources, pre-determined questions/questioning provided by physician moderators, and confirmatory patient data to provide a differential diagnosis based on a statistical likelihood of diagnosis as determined by the present system.

US6396931B1 discloses a self-contained, hand-held electronic stethoscope including built-in chest piece, speaker and visual monitor, which includes a memory containing prerecorded heart and lung sounds along with a brief description of the malady producing the sounds so that a technician may compare the actual sounds with the prerecorded sounds and observe a suggested diagnosis on the monitor.

### Statement of invention

In a first aspect there is provided an apparatus according to claim 1.

In a second aspect there is provided a computer program according to claim 9.

Some preferable features are set forth in the dependent claims.

### Brief description of drawings

The invention will now be more fully described by way of example and in conjunction with the following Figures in which:
Figure 1 shows an example user equipment;
Figure 2 shows an example of a memory structure comprising databases;
Figure 3 shows an example of database associations;
Figure 4 shows an example table of medical conditions
Figure 5 shows an example database of input prompts;
Figure 6 shows an example flow chart;
Figure 7 shows an example flow chart;
Figure 8 shows an example of associations between algorithms;
Figures 9A to 9C show example associations between algorithms;
Figure 10 shows an example flow chart;
Figure 11 shows an example database of medical advice;
Figures 12A to 12C show example user interface displays;
Figure 13 shows an example flow chart;
Figure 14 shows an example flow chart;
Figure 15 shows an example system;
Figure 16 shows an example flow chart;
Figure 17 schematically shows an example memory structure;
Figure 18 schematically shows an example user interface;
Figure 19 shows an example flow chart.

### Detailed description

Some embodiments of the invention will now be described by way of example. The disclosure relates to an apparatus and computer program for assisting a user in determining one or more medical conditions they may be suffering from, and for providing advice to the user for how to deal with the one or more medical conditions. The advice may be for example that the patient or user refers themselves to a general practitioner (GP) or physician, Pharmacist, and/or may provide advice of medication the user could take to aid in their recovery. In the examples shown the invention is computer implemented on an apparatus such as a user's mobile phone, tablet, laptop, PC etc. These devices may collectively be referred to as "user equipment".

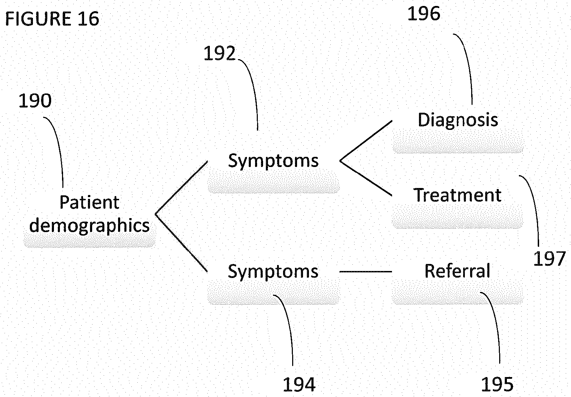

Figure 16 provides a useful overview of an embodiment. As shown at 190 patient demographics information is first retrieved from the patient. Following the top branch of the tree, medical symptom information is then retrieved from the user, as shown at 192. Following this, diagnosis of the medical condition 196 and treatment 197 may be advised to the user.

Alternatively, as shown at 194, following retrieval of symptom information at 194, a referral 195 (e.g. to a GP/Pharmacist) is advised to the user. A referral may be required, for example, if it is not possible to determine from the obtained information what the medical condition is and/or which medications should be recommended, and/or if the symptom(s) are deemed serious enough to warrant a medical consultation in person.

Embodiments enable a differential diagnosis of a user's symptoms i.e. identifying the cause of symptoms the user is suffering from, and differentiating between conditions having similar symptoms. This may enable advice to be given to the user as to whether they can self-medicate (if medication is required at all), and/or whether they should be referred to a medic such as a GPs surgery or an accident and emergency department of a hospital. Ultimately this may reduce the number of people unnecessarily visiting their GP and/or hospital, saving valuable resources.

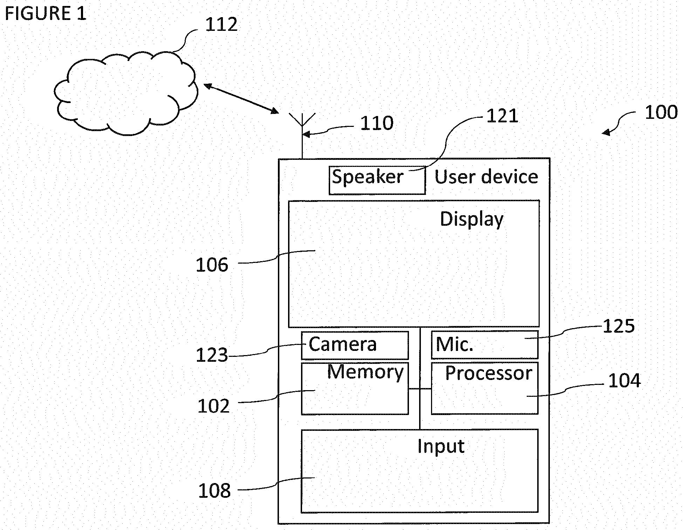

Figure 1 shows an example apparatus or user device 100 on which embodiments of the present invention may be carried out. In this example the apparatus 100 comprises a mobile phone. The apparatus comprises a memory 102 and a processor 104. The apparatus also comprises a display 106 for displaying information to a user of the apparatus 100. The apparatus further comprises input or input means 108 via which a user can input information into the apparatus. The input means 108 may comprise for example a keypad comprising one or more symbols such as numbers and letters. Where the display 106 is a touch screen display, then the input means 108 may comprise one or more portions of the touchscreen. The apparatus 100 further comprises means 110 for communication. The means for communication 110 may comprise a transmitter and receiver. Via the communication means 110 the apparatus can communicate wirelessly with one or more external apparatus such as one or more base stations, external servers, the internet etc. The external network is shown generally at 112. In this example the apparatus 100 comprises a speaker 121. The speaker 121 enables sound or audio to be output from the apparatus 100. In this example the apparatus 100 comprises a camera 123. The camera 123 enables images to be captured by the apparatus 100. In this example the camera 123 can capture still and/or motion picture images. Captured images may be stored in memory 102. In this example the apparatus 100 comprises a microphone 125. The microphone 125 enables sound or audio to be captured by the apparatus 100. Captured sounds may be stored in memory 102.

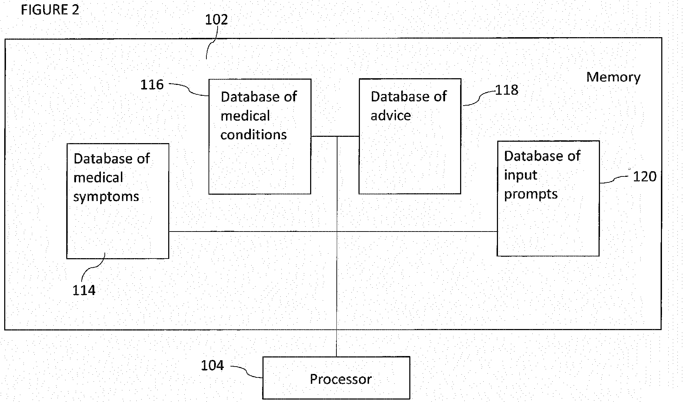

Figure 2 schematically shows a structure of the memory 102. The memory 102 stores information of medical symptoms and information of medical conditions, and associations therebetween. The memory 102 also stores information of advice that may be provided to a user. The memory 102 also stores information of input prompts designed to elicit symptoms from the user.

As shown schematically in the embodiment of Figure 2 the memory stores a plurality of databases. A first database 114 stores information of medical symptoms. Such medical symptoms may include symptoms such as cough, dehydration, nausea etc.

A second database 116 comprises a database of medical conditions. Such medical conditions may comprise for example conjunctivitis, common cold etc.

The memory further comprises a database of advice 118. This includes advice such as advising the user to make an appointment with their GP, to self-medicate, and/or advice of medications to take etc.

The memory further comprises a database of input prompts 120. These prompts may comprise for example one or more questions, or lists of selectable options that a user can select in order to supply information to the apparatus of their symptoms.

The memory 102 is communicatively connected to processor 104, such that the processor can cause information to be transmitted to and stored in the memory, and/or for retrieving information from the memory 102. The processor 104 can also perform computer operations on information stored in the memory, and generally control the user device 100.

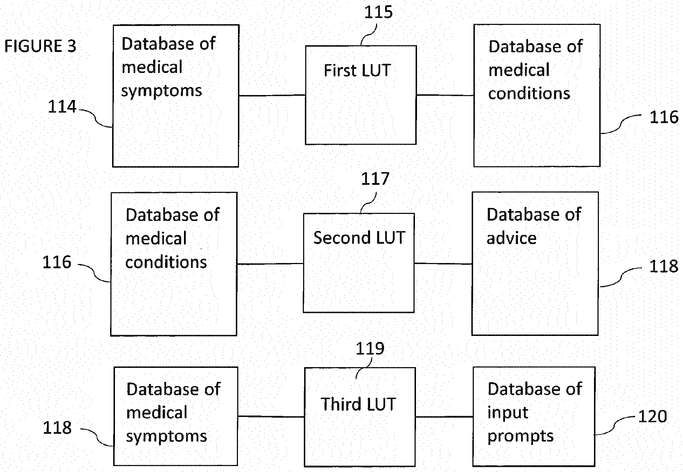

One or more of the databases are associated with each other. This enables for example one or more medical symptoms to be associated with one or more medical conditions. One or more medical symptoms and/or one or more medical conditions may be associated with one or more pieces of advice. One or more medical symptoms and/or one or more medical conditions may be associated with one or more input prompts. An exemplary structure showing such associations is shown schematically in Figure 3.

In Figure 3 the database of medical symptoms 114 is associated with the database of medical conditions 116 via a first lookup table 115. For example, the first lookup table associates a medical symptom or a combination of medical symptoms with one or more medical conditions stored in the database 116. For example the lookup table 115 may associate symptoms of cough and sore throat with a medical condition of the common cold. Certain medical symptoms or combinations of medical symptoms may be indicative of more than one medical condition. In such a case the lookup table can associate the medical symptom or combination of medical symptoms with the plurality of possible medical conditions.

The database of medical conditions 116 is associated with the database of advice 118 via second lookup table 117. For example the lookup table 117 may associate a medical condition of a common cold with advice to take paracetamol and drink water. If the medical condition is deemed more serious e.g. coughing blood, then the lookup table 117 may associate that symptom with advice to visit their GP or hospital.

A third lookup table 119 may associate the database of medical symptoms 118 with the database of input prompts 120. The database of input prompts 120 may comprise a list of symptoms and/or questions to be presented to the user. In some embodiments symptoms are displayed to a user on the display 106 of their device, which symptoms the user can then select from. In some embodiments the input prompt(s) displayed to the user are dependent upon information previously supplied by the user. For example if a user initially indicates that they have ear ache, then the lookup table 119 may associate earache with one or more possible symptoms relating to the ear, which can then be presented to the user as an input prompt. That is a filtered list of input prompts may be provided to the user, in dependence on information already supplied by the user.

The structure shown in Figure 3 is by way of example. Further associations may also be provided between the databases. For example the database of medical symptoms may also be associated with the database of advice via a lookup table, and the database of medical conditions may be associated with the database of input prompts via a lookup table, etc. One or more of the databases may also be comprised in a larger, overall database.

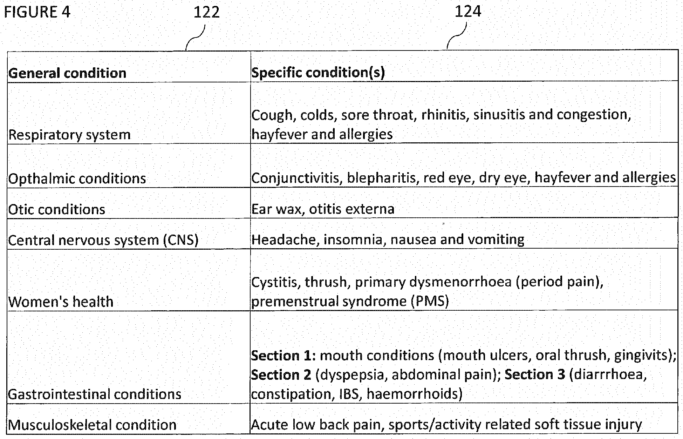

The present inventors have identified the most common conditions that are dealt with by GPs/Pharmacist. These conditions are shown in Figure 4. Each "general condition" 122 is associated with one or more "specific conditions" 124. The inventors identified the respiratory system, ophthalmic conditions, otic conditions (relating to the ear), central nervous system (CNS) conditions, women's health conditions, gastrointestinal conditions, and musculoskeletal conditions as the seven general conditions most commonly dealt with by GPs. Each general condition is associated with one or more specific conditions. For example specific conditions of the respiratory system include cough, colds, sore throats, rhinitis, sinusitis and congestion, hay fever and allergies. For example the general musculoskeletal condition is associated with the specific conditions of acute low back pain, sports/activity related soft tissue injury etc. Further relationships between general and specific medical conditions are clearly shown in Figure 4. Of course more or fewer general conditions and/or specific conditions may be provided and/or updated overtime.

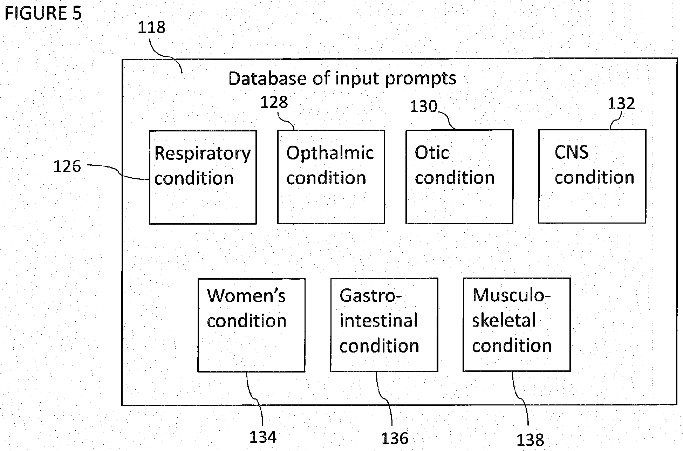

One or more of the databases stored in the memory may be structured in this fashion (i.e. as per the relationships shown in Figure 4). This is shown schematically in Figure 5 which generally shows database of medical symptoms 118. Within this database the medical symptoms may be grouped and/or segregated by type. As shown in Figure 5 respiratory system conditions are stored in memory space 126, ophthalmic conditions are stored in memory space 128, otic conditions are stored at memory space 130, CNS conditions are stored in memory space 132, women's conditions are stored in memory space 134, gastrointestinal conditions are stored in memory space 136, and musculoskeletal conditions are stored in memory space 138. Similarly, the more specific medical conditions may be nested within these overarching general medical conditions. For example within memory space 130 relating to otic conditions, there may be stored information relating to the more specific conditions of ear wax and otitis externa etc. Likewise within ophthalmic conditions 128 there may be stored information relating to the more specific conditions conjunctivitis, blepharitis, redeye, dry eye, hay fever and allergies. This structuring enables efficient retrieval of symptom information from the memory spaces. This structuring also enables efficient filtering of symptom information to be presented to the user, for example when providing a filtered list of symptoms as prompts to the user. For example if the algorithm determines that the user has an ophthalmic condition, which may be deduced from information provided by the user, then it can respond by providing input prompts relating only to ophthalmic conditions. Thus, the database of input prompts may also be segregated in the same or a similar fashion to that shown in Figure 5.

The memory 102 is further configured to store one or more algorithms. These algorithms operate to carry out the method steps as will be described in more detail below. These algorithms are stored in the form of computer program code. Generally speaking the algorithms embody, or bring in to effect, respective flowcharts aimed at obtaining symptoms from a user, and providing that user with advice. Each algorithm may be considered to constitute a portion of computer program code. Thus where different algorithms are referred to they may not necessarily exist entirely separately. That is one or more of the algorithms may exist as parts of an overall, encompassing algorithm.

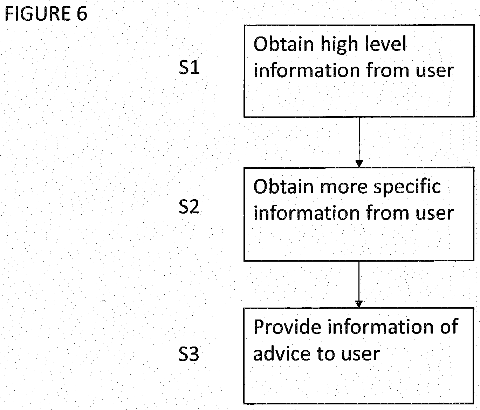

Overall steps of an example algorithm are shown in Figure 6. At step S1 the algorithm operates to obtain high-level information from a user. For example this may include causing the apparatus to provide an input prompt to the user to provide demographic information such as their gender, age etc. At step S2 the algorithm operates to obtain more specific information from the user. This may include causing the apparatus to render one or more input prompts on the display of the apparatus so as to elicit more specific information from the user, such as the medical symptoms they are experiencing. As described above, step S2 may be broken down into one or more further steps whereby filtered input prompts may be provided based upon a response given to an earlier prompt. Therefore step S2 may include two or more iterations.

Once the algorithm has obtained sufficient information from the user to enable a determination of their medical condition to be obtained, then at step S3 the algorithm causes advice to be provided to the user of how to respond to the determined medical condition. The identification of the determined medical condition may also be provided to the user. The advice may comprise for example that the user should be referred to their GP, and/or advice for self-treatment, and/or recommended medications.

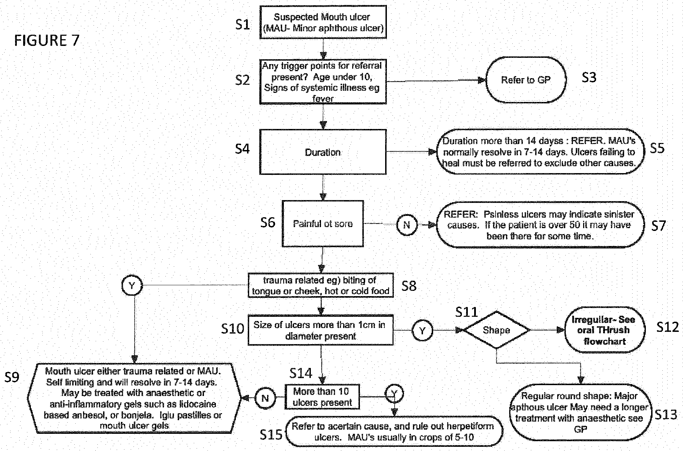

Figure 7 shows an example flowchart underpinning an algorithm.

At step S1 the algorithm has determined that the user has suspected mouth ulcers. This has been determined based upon one or more earlier questions e.g. a question of user's demographic information and/or a question relating to the general area of the symptom or symptoms.

At step S2 a determination is made as to whether there are any trigger points detected for referral to a medical professional. Such trigger conditions may include for example if the age of the user or patient is under 10 years, or signs of systemic illness, for example a fever. If the answer at step S2 is yes, then the method proceeds to step S3, where a referral is advised to the user. That is the user may be advised to obtain an appointment with their GP or see their local Pharmacist.

If the answer at step S2 is no, then the method proceeds to step S4, where the user is prompted to provide information of the duration of their symptoms. If it is determined that the duration is more than 14 days then the method proceeds to step S5 where a referral is advised to the user.

If the duration is less than 14 days then the method proceeds to step S6, where the user is prompted to provide information of whether the mouth ulcer is painful or sore. If the answer is no then the method proceeds to step S7, where the user is to be referred to their GP/Pharmacist. This is because painless ulcers may indicate sinister causes.

If the answer at step S6 is yes then the method proceeds to step S8 where the user is prompted to indicate whether the ulcer is trauma related, for example as a result of biting their tongue or cheek, or due to hot or cold food. If the answer at step S8 is yes, then the method proceeds to step S9 where the algorithm determines that the ulcer is most likely to be resolved within 7 to 14 days and that self-medication can be used, for example an anti-inflammatory gel. This advice (and indeed any other advice referred to herein) may be presented to the user on the display of their device.

If the answer at step S8 is no, then the method proceeds to step S10 where the user is prompted to insert information regarding the size of the ulcer. For example the user may be asked if the ulcer is less than or greater than 1cm in diameter. If it is determined that the ulcer is greater than 1cm in diameter then the method proceeds to step S11 where the user is prompted to input information regarding the shape of the ulcer.

If it is determined that the shape is irregular then the method proceeds to step S12. From the given information, it is determined that it is likely that the ulcer is related to oral thrush. In response the apparatus can retrieve the corresponding algorithm for suspected oral thrush diagnoses, and then continue using the oral thrush algorithm.

If it is determined from step S11 that the mouth ulcer is of a regular round shape, then it may be determined that the user should visit their GP, and a referral is advised at step S11.

If the answer at step S10 is no, then the method proceeds to step S14 where the user is prompted with a question of whether more than ten ulcers are present. If the answer is no, then the method proceeds to step S9, where self-medication is advised.

If the answer at step S14 is yes, then the method proceeds to step S15 where the user is advised to refer to their GP.

According to some embodiments when the algorithm is unable to determine the user's condition, then a referral is advised as a default.

As shown at step S12 in the flowchart of Figure 7, one flowchart/algorithm may link to another flowchart/algorithm based upon information received from the user. This dynamic and fluid association between the various algorithms enables a quicker and/or more accurate determination of the user's medical condition to be ascertained. This can speed up the process on the device and more efficiently utilise the device's computing resources.

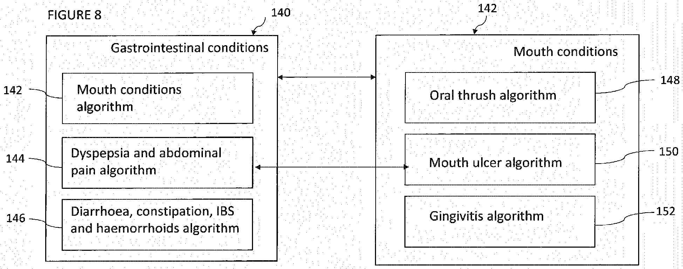

This is shown with respect to Figure 8, where a stored algorithm relating to gastrointestinal conditions is shown generally at 140. This consists of an algorithm related to mouth conditions at 142, an algorithm related to dyspepsia and abdominal pain at 144, and an algorithm related to diarrhoea, constipation, IBS and haemorrhoids at 146. Within the mouth conditions algorithm 142, there exists a mouth ulcer algorithm 148, an oral thrush algorithm 150 and a gingivitis algorithm 152.

As described with respect to Figure 7, two or more of the algorithms may be linked or associated. Dependent upon responses given by the user, the method may switch (or move) from a first algorithm to a second algorithm, and so on. For example a gastrointestinal algorithm may jump to a more specific mouth condition algorithm, as shown by arrow 154, and vice versa. A mouth ulcer algorithm 148 may jump to an oral thrush algorithm 150, as shown by arrow 156, and vice versa. A dyspepsia and abdominal pain algorithm 144 may jump to the oral thrush algorithm 150 and vice versa, as shown by arrow 158.

The decision of whether and when to switch from one algorithm to another algorithm is based upon responses provided to one or more questions or prompts.

Some symptoms may be associated with more than one medical condition. For example a certain symptom may relate to a women's condition 134 and a gastrointestinal condition 136. The decision of whether to switch algorithms may be based at least in part on determining that a symptom provided by the user is common to more than one medical condition. In some embodiments more than one algorithm can run in parallel. As per the example above, information given may indicate a women's condition or a gastrointestinal condition. Initially, both a women's condition algorithm and a gastrointestinal condition can run in parallel until a symptom or combination of symptoms is provided which enables differentiation between the two. At this point a first of the algorithms (related to the suspected condition) continues to run, whilst the other algorithm (relating to the now ruled-out condition) may be halted. This may vastly speed up the process of determining the user's condition (and accordingly utilise the computing resources more efficiently), compared with running the operations in series and requiring the user to start from the beginning for the second algorithm.

The decision of whether to switch algorithms may also take into account the number of iterations that have already been carried out with the user in order to ascertain their medical condition. For example if a user has already provided a certain number of responses and the "current" algorithm is unable to determine the user's medical condition, then the processor may cause a switching to another, different algorithm related to at least one symptom provided by the user.

Some symptoms may have a flag or alert associated within them which causes the switching from a first algorithm to a second algorithm. For example a painful or sore inner cheek symptom may have a flag associated with it that this is most likely to be related to a mouth ulcer. Therefore an initial determination may deem that a user was probably suffering from dyspepsia and abdominal pain based upon responses given, and therefore that algorithm was originally being used. However if during an iteration of the dyspepsia and abdominal pain algorithm the user indicates that they are suffering from a painful or sore inner cheek, then the processor would cause the apparatus to switch to the mouth ulcer algorithm, and continue from there. That is a determination has been made that the mouth ulcer algorithm is more relevant than the dyspepsia and abdominal pain algorithm, based on given symptom(s).

Each algorithm or flowchart may have a predetermined structure or number of steps. According to some embodiments the algorithm does not necessarily need to begin at the first step of the flowchart structure, in dependence on responses given by the user. Take for example an algorithm for abdominal pain. By way of example this algorithm may have ten predetermined input prompts, for example step S1 to step S10. Based upon input provided from the user the algorithm may begin at different starting points. For example if the user has indicated that they are an elderly man, then the next prompt from the algorithm may be different to that if the user had indicated that they were a young, pregnant woman.

This similarly applies to the switching between algorithms. For example when switching from the mouth ulcer algorithm to the oral thrush algorithm, the processor doesn't necessarily have to start at the beginning of the oral thrush algorithm, and can in fact start at a different point in the second (oral thrush) algorithm in dependence on responses already given regarding the first (mouth ulcer) algorithm.

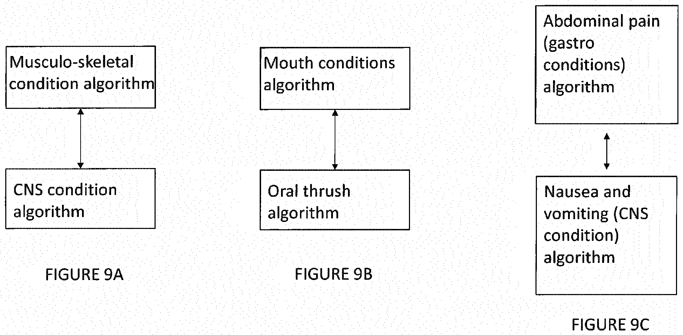

Figures 9A to 9C show some examples of switching between algorithms. Generally speaking this may be considered switching from a first algorithm to a second algorithm. The second algorithm may be considered different to the first algorithm, even though they may have at least some aspects in common. Figure 9A shows switching between a musculoskeletal condition algorithm and a CNS condition algorithm. Figure 9B shows switching between a mouth condition algorithm and an oral thrush algorithm. Figure 9C shows switching between an abdominal pain algorithm and a nausea and vomiting algorithm. The double headed arrows show that the switching can take place in either direction.

According to some embodiments it is determined whether a user should be referred for medical attention e.g. to see their GP or physician. According to some embodiments a referral is advised to the user in response to receiving one or more trigger conditions. These may be termed referral trigger conditions. A referral trigger condition can be a medical symptom and/or demographic information of the user. For example a trigger condition may be a user's age or a specific medical symptom of the user e.g. coughing blood. To this end certain symptoms and/or other information have associated therewith a flag or alert, the flag or alert identifying the respective symptom and/or information as a trigger condition for referral. This is shown for example in the flowchart of Figure 7, where input of a user's age under 10 and/or presence of a fever causes the algorithm to advise a referral to the GP/Pharmacist. Likewise at step S6 the presence of painless ulcers is considered a referral trigger condition. In some embodiments the presence of a referral trigger causes the algorithm to stop providing the user with further prompts, and instead provides the referral advice to the user. That is the algorithm does not continue to present the user with further input prompts once the referral condition has been met. Again this efficiently utilises computing resources since the entire algorithm may not need to be run through in its entirety, as well as efficiently utilising the user's time and ensures that they obtain the necessary referral as soon as possible.

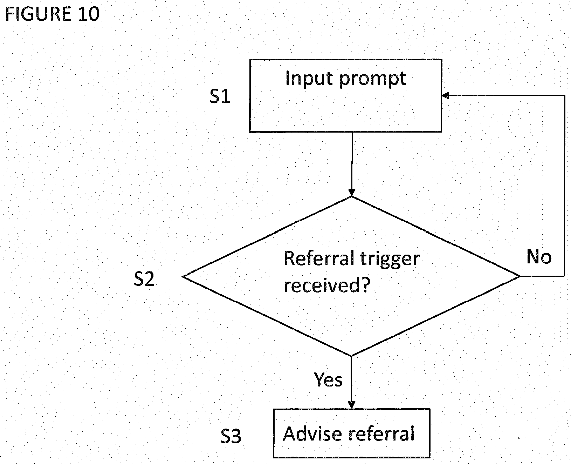

This is described in more detail with respect to Figure 10. At step S1 the user is presented with an input prompt which they can respond to.

Once the user has responded then the method continues to step S2 where a determination is made as to whether a referral trigger has been received. If the answer at step S2 is no, then the algorithm loops back to step S1 where the user continues to be prompted to provide more information. If the answer at step S2 is yes, then the method continues to step S3, where a referral is advised.

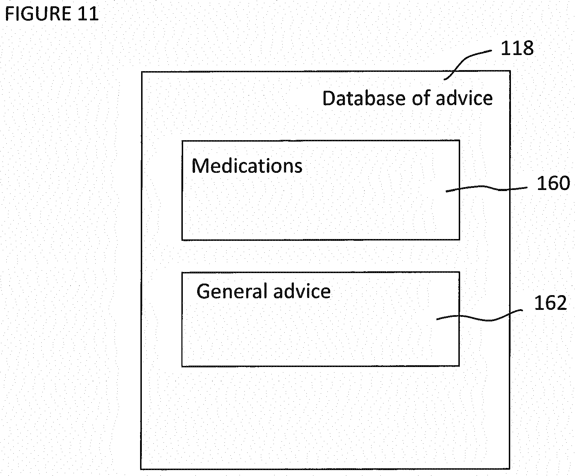

Figure 11 shows a database of advice 118 in more detail. In this embodiment the advice database comprises medication advice 160 and general advice 162. The medications advice 160 comprises advice of medications that the user could take which may improve their condition. This may include medication such as paracetamol, Gaviscon^{®}, cough syrup etc. The general advice 162 may be provided alternatively and/or in addition to the medication advice. The general advice may comprise advice to see your GP i.e. referral advice. The general advice may comprise other advice such as to drink lots of water, take a rehydration sachet, rest for 24 hours etc.

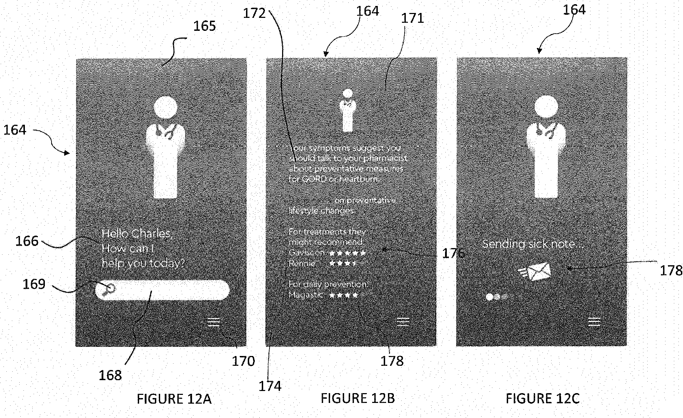

Figures 12A to 12C show some example screens of a user interface 164 according to embodiments.

Figure 12A shows a first screen 165. This first screen may be a home screen e.g. a screen that is displayed to a user when they open the application. The first screen provides a prompt 166 to a user, in this case a question "how can I help you today?". There is then an input area 168 enabling a user to input information. For example this may include information of their symptoms. In some embodiments this can be entered in a free-form e.g. with the user typing in their symptoms. Voice recognition software may also be employed, enabling the user to input their symptoms by speaking in to the device. A search icon in the form of magnifying glass 169 is also provided. **In** some embodiments when a user selects this option then further options are provided to a user. For example a further screen may be displayed showing a list of symptoms that a user can choose from, or other prompts aimed at eliciting a response from the user.

A settings or options icon 170 is also provided. When a user selects icon 170 then one or more further pieces of information may be provided to the user. Via icon 170 a user may carry out tasks such as changing account settings etc.

After a user has input sufficient information enabling the algorithm or algorithms to determine a medical condition of a user, then a screen such as that shown in Figure 12B may be provided. This screen 171 comprises advice in region 172 that the user should talk to their pharmacist about preventative measures for gastro-oesophageal reflux disease (GORD) or heart burn.

In region 174 of screen 171 recommended treatments are provided, in this case Gaviscon^{®} and Rennie^{®}. It should further be noted that these recommended treatments are given a ranking based on their deemed effectiveness for the given condition. This is shown at 176. In this case Gaviscon^{®} is rated at 5 out of 5 and Rennie is rated at 3 ½ out of 5^{®}.

Region 178 furthermore provides advice for daily preventative measures that can be taken. In this case Magastic^{®} is recommended for daily and/or ongoing treatment. This is further given a ranking, in this case 4 out of 5.

Another aspect is the ability to generate and/or send a sick note to a user's employer/workplace/educational establishment etc. as appropriate. In some embodiments the algorithm can determine whether the user's symptoms or combination of symptoms will make them too unwell and/or contagious to attend their workplace. When it is determined that a user is too ill to attend their workplace then one or more options may be provided. In one option, the user is given an option asking whether they would like a sick note to be generated. In another option a sick note may be automatically generated. An option may further be provided asking the user to confirm whether they want the sick note to be sent to their employer. If the user confirms that this is the case then a sick note is sent. Alternatively the sick note may be automatically sent to the employer. In some embodiments a sick note form is sent to the user enabling that user to fill in the sick note form and independently supply it to their employer as appropriate.

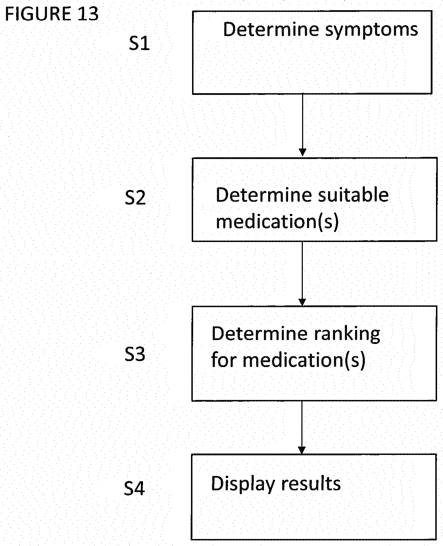

A process for determining and providing medication rankings to a user is shown in Figure 13. At step S1 the user's symptoms are determined. This may be carried out as explained in the embodiments above. At step S2 suitable medications are determined. This may comprise one or more suitable medications. For example if the user has a headache then a suitable medication may be paracetamol. At step S3 a ranking for each of the medications is provided. This ranking may be provided in any form, for example the star rankings as shown in Figure 12B. The rankings may be determined in a dynamic fashion dependent upon the information provided by the user. The rankings may vary depending upon the demographic information of the user of e.g. age, gender, and/or the symptoms provided. In some embodiments the ranking of a medication or treatment is based upon an average value for that medication or treatment for the combination of symptoms provided. By way of example, say a user's symptoms have been identified as a headache and a sore throat. Paracetamol may have a 5 out of 5 ranking of effectiveness for headaches, and a ranking of 3 out of 5 for sore throats. Therefore overall paracetamol may be given a ranking of 4 out of 5 for treating the user's symptoms.

At step S4 the results are displayed to the user, as shown for example in Figure 12B.

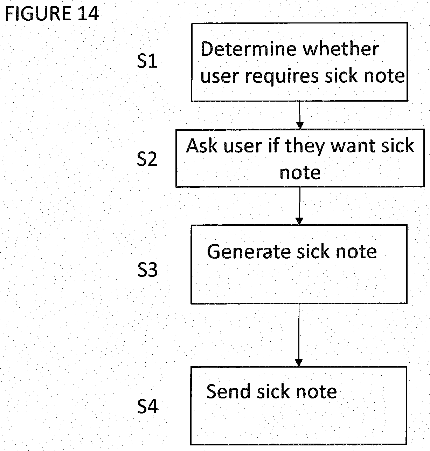

Steps for generating a sick note are shown in Figure 14. At step S1 a determination is made as to whether the user requires a sick note. As explained above this is based upon the information provided by the user. Optionally, at step S2 the user is asked if they want a sick note. At step S3 the sick note is generated. Of course step S3 only occurs if the user confirms that they want the sick note. At step S4 the sick note is sent. For example the sick note is sent to a designated person at the user's workplace. Such information of the designated person may be stored within the application, so that this information does not have to be re-entered by the user each time they need to send a sick note. This process may significantly reduce resource consumption at GP practices, since the generation of sick notes can be a large part of their workload. For an employer this is advantageous since sick notes can easily be stored electronically and kept on records. It also facilitates tracking of the number of sick notes a user has sent.

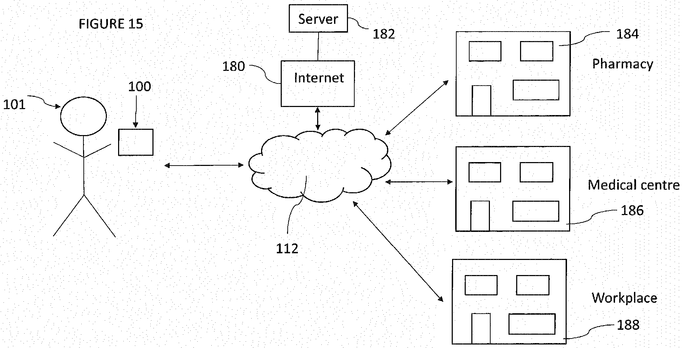

Figure 15 shows some further aspects. A user 101 is shown operating their user device 100 e.g. mobile phone. The user has downloaded an application or "app" embodying the invention. Alternatively the program or application is hosted on a website on internet 180, which the user can connect to. Accordingly via user device 100 the user 101 can input their information and symptoms and receive an output in the form of medical advice, as described above. Via network 112 the user can connect to, for example the Internet 180.

Via the Internet the device 100 may connect to one or more servers and/or stores, enabling the application to be downloaded in the first place and/or updates to be subsequently received. The user device 100 can also upload received information to one or more associated servers 182. As well as receiving information from the server 182, the user device 100 can also upload information to the server 182. The server 182 may collate information from multiple users over time. This may enable the system to learn over time based on feedback received from multiple users. Updates can then be sent to the mobile device 100 to include the new information. For example the updates may include information such as new symptoms and medical conditions and their associations, as well as updates to recommended treatments. In some embodiments the program is downloaded in its entirety to the mobile device. That is all information necessary to run the program is stored on the device. Thereafter the application can be used without an internet connection. In some embodiments a reduced amount of functionality is downloaded to the mobile device, with at least some functionality remaining at the server end 182. For example one or more of the above described databases and look up tables may be stored at the server. Information is input at the mobile device 100 and sent to server 182, where it can be processed and the advice generated at the server. The advice is then sent back to the mobile device 100 where it is displayed to user 101. In such embodiments the server comprises a memory and a processor which operate in an equivalent manner as described above. Such distributed architecture reduces processing and memory burden on the mobile device.

Also shown in Figure 15 is a pharmacy 184. Using location services such as GPS, information on the proximity of one or more pharmacies can be provided to the user. In some embodiments the user device can be caused to send one or more queries to the pharmacy 184. Referring back to the example of Figure 12B recommended treatments are Gaviscon^{®} and Rennie^{®}. The processor may cause a query to be sent to the pharmacy 184 to stock-check that these products are in store. This may potentially save a wasted visit to the pharmacy, and furthermore helps the pharmacy with maintaining correct levels of stock. In some embodiments the device may send a query to the pharmacy which causes a machine at the pharmacy to dispense the medications required, ready to be picked up by the user. In some embodiments the pharmacy 184 may be an unmanned pharmacy e.g. a kiosk or booth. The mobile device 100 may send a query to the kiosk or booth causing the kiosk or booth to prepare the required medications ready for collection. To this end a code or password may be provided to the user device 100, which code or password can then be used at the kiosk or booth to retrieve the dispensed medicines.

The user device 100 may also connect via network 112 to medical centre 186. The medical centre may be for example a GP surgery, Accident and Emergency department in a hospital etc. Where the advice provided to the user is to be referred to a GP, then this connection enables an appointment to be made with the GP. In some embodiments a request for a doctor's appointment is automatically made with the GP surgery when a GP referral is recommended. In some embodiments the process is semi-automatic, for example a link may be provided on the screen with a prompt such as "would you like to make an appointment with your GP?". When a user clicks on the link or confirms "yes" then a message is sent to the medical centre 186 requesting an appointment. This automatic or semiautomatic requesting of GP appointment is convenient to the user, and may also reduce load on system resources. For example it reduces the number of steps required on behalf of the user to arrange a doctor's appointment, as well as enabling systems at the medical-centre end to efficiently schedule appointment and reduces the amount of time administration staff need to spend on the telephone and/or computers entering details of the user and/or their symptoms etc., since some of these may be automatically uploaded to the medical centre computers and/or servers. This can vastly reduce the amount of time that GPs need to spend with each patient, since when the patient arrives the GP will already have access to information regarding their symptoms.

The user device 100 can also connect via network 112 to workplace 188 to send a sick note as previously described.

A user can save one or more pieces of information in their account settings. For example a user can save settings and contact details of their local or preferred pharmacy (or have a setting that causes the device to find the nearest pharmacy). A user can also save information of their GP and associated relevant contact information. A user can also save information of their workplace and associated contact information. This enables multiple tasks to be carried out in relatively few steps, saving computing resources. For example once a user's illness is determined, the device can cause a query to be sent to one or more of: the GP for an appointment to be made; the pharmacy for medication(s) to be prepared and/or delivered; the workplace for the relevant person to be informed of the sickness and/or for a sick note to be sent. This may be carried out by the user confirming that they want each step to be carried out (e.g. one click or confirmation for each), or in another example one click or confirmation for all of these actions to be carried out.

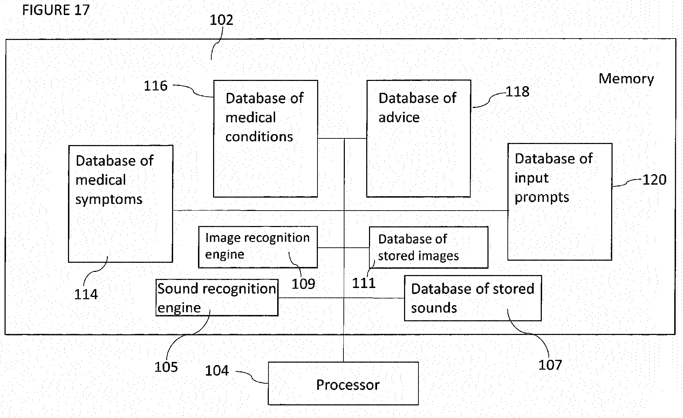

Figure 17 shows an example based on the example of Figure 2, with some further features. For conciseness, explanation of aspects in common with Figure 2 are not repeated. In the example of Figure 17 the memory 102 stores a database of stored sounds 107, and/or information of one or more sounds. The stored sounds are associated with one or more illnesses or ailments. Different sounds may be representative of different illnesses, and/or representative of different types of a same illness or symptom. The stored sounds include a sound of a cough. There may be different stored sounds associated with different types of cough. For example a first stored sound may be associated with a chesty cough, and a second stored sound may be associated with a dry cough. Other example sounds may be stored, in addition to the cough sound. For example there may be stored sounds each representing a different breathing condition, or a different ear condition (e.g. tinnitus). The stored sounds may be played to a user, for example in response to a request from a user. The played stored sounds may then be used by the user to help decide or distinguish what kind or type of illness they have.

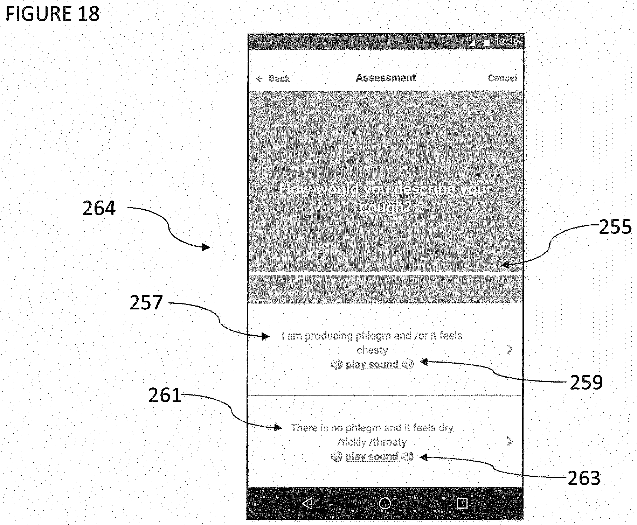

An example is shown with respect to Figure 18. This shows a user interface 264. On the user interface 264 a prompt 255 is provided asking a user "How would you describe your cough?". A first selectable option is shown at 257, "I am producing phlegm and/or it feels chesty". To help a user determine what this type of cough sounds like they can hear a preview of such a cough by selecting the "play sound" icon 259. A second selectable option is shown at 261, "There is no phlegm and it feels dry/tickly/throaty". To help a user determine what this type of cough sounds like they can hear a preview of such a cough by selecting the "play sound" icon 263. This may help a user correctly diagnose their illness, and prevent or reduce the chances of mis-diagnosis.

As shown in Figure 17 the memory also stores a sound recognition engine or unit 105. The sound recognition engine may comprise an acoustic model which stores representations and/or information of different sounds. The acoustic model may comprise the database of stored sounds 107. The sound recognition engine 105 may receive input comprising a sound from a user, and in conjunction with processor 104 can classify that received sound as a medical symptom e.g. chesty cough, strained breathing, blocked nose etc. The input from the user may be provided in response to a prompt from the apparatus to the user to provide an example of their symptom e.g. "Please cough now". In some examples the acoustic model is updateable over time e.g. via a network update. The acoustic model may also learn and update using information received at the apparatus 100. Thus the sound recognition engine 105 may comprise, and/or be in communication with, a machine learning or deep learning algorithm. Therefore over time the acoustic model can update and improve its recognition of sounds, and the apparatus 100 and system can improve or make more accurate its association between symptoms (e.g. in the form of received sounds) and medical conditions.

In the example of Figure 17 the memory 102 also stores a database of stored images 111 or information of images. The stored images 112 may be selectively provided on the user interface of the apparatus 100, to assist a user with a diagnosis. For example an input prompt may state "What colour is the bruise?", and an accompanying selectable option may be "Show examples of bruises". Other images may, for example, help a user in diagnosing eye conditions e.g. red-eye, and/or different types of spots or rashes.

In the example of Figure 17 an image recognition engine or unit 109 is shown. The image recognition engine 109 may comprise an image model which stores data or information of one or more images. The image model may comprise the database of stored images 111. The image recognition engine 109 may receive input comprising an image from a user, e.g. captured on camera 123, and in conjunction with processor 104 can classify that received image as a medical symptom e.g. red eye, eczema etc. The input from the user may be provided in response to a prompt from the apparatus to the user to provide an example of their symptom e.g. "Please take a photo of your rash". In some examples the image model is updateable over time e.g. via a network update. The image model may also learn and update using information received at the apparatus 100. Thus the image recognition engine 109 may comprise, and/or be in communication with, a machine learning or deep learning algorithm. Therefore over time the image model can update and improve its recognition of images, and the apparatus 100 and system can improve or make more accurate its association between symptoms (e.g. in the form of received images) and medical conditions.

Such examples may be particularly useful where a patient has difficulty in explaining their symptoms, for example if they are young or old or have difficulty understanding the prompts. Accordingly such examples may improve diagnosis of medical conditions. Such examples may also decrease the number of input prompts required in order to ascertain a diagnosis of a user. For example the acoustic model may be able to diagnose a type of cough following a single input prompt to a user to provide an example of their cough, where for example five or more question and answer prompts may have been required in order to diagnose the type of cough. Similarly a rash may be more quickly identified using the image recognition engine 109. This therefore potentially reduces the amount of signalling required within the apparatus 100 and/or with network 112 associated with providing question and input prompts.

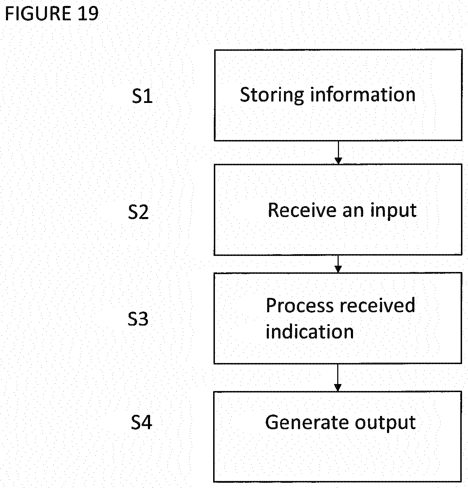

An example method is shown with respect to Figure 19.

At step S1 information is stored in a memory of an apparatus. This information comprises information of a plurality of medical symptoms. The method further comprises storing in the memory associations between one or more of the medical symptoms and information of one or more medical conditions.

At step S2 an input is received. The input is received from a user via a user interface of the apparatus, the input comprising an indication of one or more medical symptoms of the user.

At step S3 the received indication is processed, so as to determine a medical condition associated with the indication of one or more medical symptoms.

At step S4 an output is generated and displayed on a display of the apparatus. The output comprises advice to the user of how to respond to the medical condition.

It will be understood that the processor or processing system or circuitry referred to herein may in practice be provided by a single chip or integrated circuit or plural chips or integrated circuits, optionally provided as a chipset, an application-specific integrated circuit (ASIC), field-programmable gate array (FPGA), digital signal processor (DSP), graphics processing units (GPUs), etc. The chip or chips may comprise circuitry (as well as possibly firmware) for embodying at least one or more of a data processor or processors, a digital signal processor or processors, baseband circuitry and radio frequency circuitry, which are configurable so as to operate in accordance with the exemplary embodiments. In this regard, the exemplary embodiments may be implemented at least in part by computer software stored in (non-transitory) memory and executable by the processor, or by hardware, or by a combination of tangibly stored software and hardware (and tangibly stored firmware).

Reference is made herein to data storage for storing data. This may be provided by a single device or by plural devices. Suitable devices include for example a hard disk and non-volatile semiconductor memory.

The examples described herein are to be understood as illustrative examples of embodiments of the invention. The scope of the invention is defined in the claims.

## Claims

1. An apparatus (100)comprising:
a memory (102);
a processor (104);
the memory (102) storing information of a plurality of medical symptoms, the memory (102) further storing associations between one or more of the medical symptoms and information of one or more medical conditions;
the memory (102) storing information of at least first and second input prompts to be displayed on a user interface of the apparatus, so as to elicit one or more medical symptoms from the user, a content of the second input prompt being determined in dependence on a response received to the first input prompt;
the apparatus (100) configured to receive an input from a user (101) via a user interface of the apparatus, the input comprising an indication of one or more medical symptoms of the user;
the processor (104) configured to process the received indication of one or more medical symptoms of the user, to determine a medical condition associated with the indication of one or more medical symptoms; and
the processor (104) configured to generate an output to the user on a display (106) of the apparatus, the output comprising advice to the user of how to respond to the medical condition;
wherein the apparatus (100)stores information of one or more sounds in the memory (102), each sound associated with a symptom of the plurality of medical symptoms, wherein the information of one or more sounds comprises information of one or more cough sounds; and
the apparatus (100) configured to play one or more of the sounds stored in the memory (102) so as to assist a user in indicating one or more symptoms; and
the memory (102) storing a plurality of algorithms (140, 142), each of the algorithms associated with a corresponding medical condition, the first input prompt comprised in a first algorithm (140) associated with a first medical condition, the second input prompt comprised in a second algorithm (142) associated with a second medical condition, the apparatus (100) configured to switch from the first algorithm to the second algorithm in response to determining that the second algorithm is more applicable to the user's symptoms than the first algorithm.

2. The apparatus (100) as set forth in claim 1, the apparatus (100) configured to provide information to the user of an identification of the medical condition as part of the output.

3. The apparatus (100) as set forth in claim 1 or claim 2, the memory (102) storing information of the advice to the user of how to respond to the medical condition, and the memory (102) storing associations between the one or more medical conditions and the advice to the user of how to respond to the medical condition.

4. The apparatus (100) as set forth in any of claims 1 to 3, the memory (102) storing information of one or more products recommended to treat the determined medical condition, the apparatus (100) configured to indicate one or more of the products as the advice or part of the advice, the apparatus (100) configured to determine a ranking of the one or more recommended products, and to display the ranking to the user, the ranking being determined based upon the received one or more medical symptoms.

5. The apparatus (100) as set forth in any of claims 1 to 4, wherein the advice to the user comprises advice that a referral to a medical practitioner should be sought, the memory (102) storing one or more trigger conditions for recommending the referral, the one or more trigger conditions comprising one or more symptoms or combination of symptoms.

6. The apparatus (100) as set forth in any of claims 1 to 5, the apparatus (100) configured to receive sound input at the apparatus (100) from a user, and the apparatus (100) configured to use an acoustic model to associate the sound input with the information of one or more sounds stored in the memory, so as to associate the received sound input with a symptom of the plurality of medical symptoms.

7. The apparatus (100) as set forth in any of claims 1 to 6, the apparatus (100) configured to receive an image input at the apparatus from a user, and the apparatus configured to use an image recognition engine to associate the received image with information of one or more images stored in the memory, so as to associate the received image with a symptom of the plurality of medical symptoms.

8. The apparatus (100) as set forth in any of claims 1 to 7, wherein the information of one or more sounds comprises a first sound associated with a chesty cough and a second sound associated with a dry cough.

9. A computer program comprising computer executable instructions which when run on one or more processors performs:
storing in a memory (102) of an apparatus information of a plurality of medical symptoms, and further storing in the memory associations between one or more of the medical symptoms and information of one or more medical conditions;
storing information of at least first and second input prompts to be displayed on a user interface of the apparatus, so as to elicit one or more medical symptoms from the user, a content of the second input prompt being determined in dependence on a response received to the first input prompt;
receiving an input from a user (101) via a user interface of the apparatus, the input comprising an indication of one or more medical symptoms of the user;
processing, using a processor (104), the received indication of one or more medical symptoms of the user, to determine a medical condition associated with the indication of one or more medical symptoms; and
generating an output to the user on a display (106) of the apparatus, the output comprising advice to the user of how to respond to the medical condition; **characterised in that** the method comprises
storing information of one or more sounds in the memory (102), each sound associated with a symptom of the plurality of medical symptoms, wherein the information of one or more sounds comprises information of one or more cough sounds;
playing one or more of the sounds stored in the memory (102) so as to assist a user in indicating one or more symptoms;
storing a plurality of algorithms (140, 142), each of the algorithms associated with a corresponding medical condition, the first input prompt comprised in a first algorithm (140) associated with a first medical condition, the second input prompt comprised in a second algorithm (142) associated with a second medical condition, and switching from the first algorithm to the second algorithm in response to determining that the second algorithm is more applicable to the user's symptoms than the first algorithm.

## Patentansprüche

1. Gerät (100), umfassend:
einen Speicher (102);
einen Prozessor (104);
der Speicher (102) Informationen über eine Vielzahl von medizinischen Symptomen speichert, wobei der Speicher (102) ferner Assoziationen zwischen einem oder mehreren der medizinischen Symptome und Informationen über einen oder mehrere medizinische Zustände speichert;
der Speicher (102) Informationen von mindestens ersten und zweiten Eingabeaufforderungen speichert, die auf einer Benutzerschnittstelle des Geräts angezeigt werden sollen, um dem Benutzer ein oder mehrere medizinische Symptome zu entlocken, wobei ein Inhalt der zweiten Eingabeaufforderung in Abhängigkeit von einer auf die erste Eingabeaufforderung empfangenen Antwort bestimmt wird;
das Gerät (100) konfiguriert ist, um eine Eingabe von einem Benutzer (101) über eine Benutzerschnittstelle des Geräts zu empfangen, wobei die Eingabe eine Angabe eines oder mehrerer medizinischer Symptome des Benutzers umfasst;
der Prozessor (104) konfiguriert ist, um die empfangene Angabe von einem oder mehreren medizinischen Symptomen des Benutzers zu verarbeiten, um einen medizinischen Zustand zu bestimmen, der mit der Angabe von einem oder mehreren medizinischen Symptomen verbunden ist; und
der Prozessor (104) konfiguriert ist, um eine Ausgabe an den Benutzer auf einer Anzeige (106) des Geräts zu erzeugen, wobei die Ausgabe Ratschläge an den Benutzer umfasst, wie er auf den medizinischen Zustand reagieren soll;
wobei das Gerät (100) Informationen über ein oder mehrere Geräusche in dem Speicher (102) speichert, wobei jedes Geräusch mit einem Symptom der Vielzahl von medizinischen Symptomen verbunden ist, wobei die Informationen über ein oder mehrere Geräusche Informationen über ein oder mehrere Hustengeräusche umfassen; und
das Gerät (100) konfiguriert ist, um einen oder mehrere der in dem Speicher (102) gespeicherten Geräusche abzuspielen, um einen Benutzer bei der Angabe eines oder mehrerer Symptome zu unterstützen; und
der Speicher (102) eine Vielzahl von Algorithmen (140, 142) speichert, wobei jeder der Algorithmen einem entsprechenden medizinischen Zustand zugeordnet ist, die erste Eingabeaufforderung einen ersten Algorithmus (140) umfasst, der einem ersten medizinischen Zustand zugeordnet ist, die zweite Eingabeaufforderung einen zweiten Algorithmus (142) umfasst, der einem zweiten medizinischen Zustand zugeordnet ist, und die Vorrichtung (100) konfiguriert ist, um in Reaktion auf das Bestimmen, dass der zweite Algorithmus besser auf die Symptome des Benutzers anwendbar ist als der erste Algorithmus, von dem ersten Algorithmus zu dem zweiten Algorithmus zu wechseln.

2. Gerät (100) nach Anspruch 1, wobei das Gerät (100) konfiguriert ist, um dem Benutzer Informationen über eine Identifizierung des medizinischen Zustands als Teil der Ausgabe bereitzustellen.

3. Gerät (100) nach Anspruch 1 oder 2, wobei der Speicher (102) Informationen über den Ratschlag an den Benutzer, wie er auf den medizinischen Zustand reagieren soll, speichert, und der Speicher (102) Assoziationen zwischen dem einen oder den mehreren medizinischen Zuständen und dem Ratschlag an den Benutzer, wie er auf den medizinischen Zustand reagieren soll, speichert.

4. Gerät (100) nach einem der Ansprüche 1 bis 3, wobei der Speicher (102) Informationen über ein oder mehrere Produkte speichert, die zur Behandlung des bestimmten medizinischen Zustands empfohlen werden, wobei das Gerät (100) konfiguriert ist, um eines oder mehrere der Produkte als Empfehlung oder Teil der Empfehlung anzugeben, wobei das Gerät (100) konfiguriert ist, um eine Rangfolge des einen oder der mehreren empfohlenen Produkte zu bestimmen und dem Benutzer die Rangfolge anzuzeigen, wobei die Rangfolge basierend auf dem empfangenen einen oder mehreren medizinischen Symptomen bestimmt wird.

5. Gerät (100) nach einem der Ansprüche 1 bis 4, wobei der Ratschlag an den Benutzer einen Ratschlag umfasst, dass eine Überweisung an einen Arzt erfolgen sollte, wobei der Speicher (102) eine oder mehrere Auslösebedingungen für die Empfehlung der Überweisung speichert, wobei die eine oder mehreren Auslösebedingungen ein oder mehrere Symptome oder eine Kombination von Symptomen umfassen.

6. Gerät (100) nach einem der Ansprüche 1 bis 5, wobei das Gerät (100) konfiguriert ist, um eine Schalleingabe an dem Gerät (100) von einem Benutzer zu empfangen, und das Gerät (100) konfiguriert ist, um ein akustisches Modell zu verwenden, um die Geräuscheingabe mit den Informationen eines oder mehrerer Geräusche, die in dem Speicher gespeichert sind, zu verknüpfen, um so die empfangene Geräuscheingabe mit einem Symptom der Vielzahl von medizinischen Symptomen zu verknüpfen.

7. Gerät (100) nach einem der Ansprüche 1 bis 6, wobei das Gerät (100) konfiguriert ist, um eine Bildeingabe an dem Gerät von einem Benutzer zu empfangen, und wobei das Gerät konfiguriert ist, um eine Bilderkennungsmaschine zu verwenden, um das empfangene Bild mit Informationen von einem oder mehreren Bildern, die in dem Speicher gespeichert sind, zu verknüpfen, um das empfangene Bild mit einem Symptom der Vielzahl von medizinischen Symptomen zu verknüpfen.

8. Gerät (100) nach einem der Ansprüche 1 bis 7, wobei die Informationen eines oder mehrerer Geräusche ein erstes Geräusch umfassen, das einem Reizhusten zugeordnet ist, und ein zweites Geräusch, das einem trockenen Husten zugeordnet ist.

9. Computerprogramm, umfassend computerausführbare Anweisungen, die, wenn sie auf einem oder mehreren Prozessoren ausgeführt werden, Folgendes bewirken:
Speichern von Informationen über eine Vielzahl von medizinischen Symptomen in einem Speicher (102) eines Geräts, und ferner Speichern von Assoziationen zwischen einem oder mehreren der medizinischen Symptome und Informationen über einen oder mehrere medizinische Zustände in dem Speicher;
Speichern von Informationen über mindestens erste und zweite Eingabeaufforderungen, die auf einer Benutzerschnittstelle des Geräts angezeigt werden sollen, um dem Benutzer ein oder mehrere medizinische Symptome zu entlocken, wobei ein Inhalt der zweiten Eingabeaufforderung in Abhängigkeit von einer auf die erste Eingabeaufforderung empfangenen Antwort bestimmt wird;
Empfangen einer Eingabe von einem Benutzer (101) über eine Benutzerschnittstelle des Geräts, wobei die Eingabe eine Angabe von einem oder mehreren medizinischen Symptomen des Benutzers umfasst;
Verarbeiten, unter Verwendung eines Prozessors (104), der empfangenen Angabe eines oder mehrerer medizinischer Symptome des Benutzers, um einen medizinischen Zustand zu bestimmen, der mit der Angabe eines oder mehrerer medizinischer Symptome verbunden ist; und
Erzeugen einer Ausgabe an den Benutzer auf einer Anzeige (106) des Geräts, wobei die Ausgabe Ratschläge an den Benutzer umfasst, wie er auf den medizinischen Zustand reagieren soll; **dadurch gekennzeichnet, dass** das Verfahren Folgendes umfasst:
Speichern von Informationen über ein oder mehrere Geräusche in dem Speicher (102), wobei jedes Geräusch mit einem Symptom aus der Vielzahl der medizinischen Symptome verbunden ist, wobei die Informationen über ein oder mehrere Geräusche Informationen über ein oder mehrere Hustengeräusche umfassen;
Abspielen eines oder mehrerer der im Speicher (102) gespeicherten Geräusche, um einen Benutzer bei der Angabe eines oder mehrerer Symptome zu unterstützen;
Speichern einer Vielzahl von Algorithmen (140, 142), wobei jeder der Algorithmen einem entsprechenden medizinischen Zustand zugeordnet ist, wobei die erste Eingabeaufforderung einen ersten Algorithmus (140) umfasst, der einem ersten medizinischen Zustand zugeordnet ist, wobei die zweite Eingabeaufforderung einen zweiten Algorithmus (142) umfasst, der einem zweiten medizinischen Zustand zugeordnet ist, und Umschalten von dem ersten Algorithmus zu dem zweiten Algorithmus in Reaktion auf das Bestimmen, dass der zweite Algorithmus besser auf die Symptome des Benutzers anwendbar ist als der erste Algorithmus.

## Revendications

1. Appareil (100) comprenant :
une mémoire (102) ;
un processeur (104) ;
la mémoire (102) stockant des informations sur une pluralité de symptômes médicaux, la mémoire (102) stockant en outre des associations entre un ou plusieurs des symptômes médicaux et des informations sur une ou plusieurs pathologies médicales ;
la mémoire (102) stockant des informations sur au moins des première et seconde invites d'entrée à afficher sur une interface utilisateur de l'appareil, de manière à susciter un ou plusieurs symptômes médicaux chez l'utilisateur, un contenu de la seconde invite d'entrée étant déterminé en fonction d'une réponse reçue à la première invite d'entrée ;
l'appareil (100) étant configuré pour recevoir une entrée d'un utilisateur (101) via une interface utilisateur de l'appareil, l'entrée comprenant une indication d'un ou de plusieurs symptômes médicaux de l'utilisateur ;
le processeur (104) étant configuré pour traiter l'indication reçue d'un ou de plusieurs symptômes médicaux de l'utilisateur, pour déterminer une pathologie médicale associée à l'indication d'un ou de plusieurs symptômes médicaux ; et
le processeur (104) étant configuré pour générer une sortie à l'utilisateur sur un écran (106) de l'appareil, la sortie comprenant un conseil à l'utilisateur sur la manière de réagir à la pathologie médicale ;
dans lequel l'appareil (100) stocke des informations sur un ou plusieurs sons dans la mémoire (102), chaque son étant associé à un symptôme de la pluralité de symptômes médicaux, dans lequel les informations sur un ou plusieurs sons comprennent des informations sur un ou plusieurs sons de toux ; et
l'appareil (100) étant configuré pour jouer un ou plusieurs des sons stockés dans la mémoire (102) afin d'aider un utilisateur à indiquer un ou plusieurs symptômes ; et
la mémoire (102) stockant une pluralité d'algorithmes (140, 142), chacun des algorithmes étant associé à une pathologie médicale correspondante, la première invite d'entrée étant comprise dans un premier algorithme (140) associé à une première pathologie médicale, la seconde invite d'entrée étant comprise dans un second algorithme (142) associé à une seconde pathologie médicale, l'appareil (100) étant configuré pour passer du premier algorithme au second algorithme en réponse à la détermination que le second algorithme est plus applicable aux symptômes de l'utilisateur que le premier algorithme.

2. Appareil (100) selon la revendication 1, l'appareil (100) étant configuré pour fournir des informations à l'utilisateur sur une identification de la pathologie médicale en tant que partie de la sortie.

3. Appareil (100) selon dans la revendication 1 ou la revendication 2, la mémoire (102) stockant des informations sur le conseil à l'utilisateur sur la manière de réagir à la pathologie médicale, et la mémoire (102) stockant des associations entre l'une ou les plusieurs pathologies médicales et le conseil à l'utilisateur sur la manière de réagir à la pathologie médicale.

4. Appareil (100) selon l'une quelconque des revendications 1 à 3, la mémoire (102) stockant des informations sur un ou plusieurs produits recommandés pour traiter la pathologie médicale déterminée, l'appareil (100) étant configuré pour indiquer un ou plusieurs des produits comme conseil ou partie du conseil, l'appareil (100) étant configuré pour déterminer un classement de l'un ou des plusieurs produits recommandés, et pour afficher le classement à l'utilisateur, le classement étant déterminé sur la base de l'un ou des plusieurs symptômes médicaux reçus.

5. Appareil (100) selon l'une quelconque des revendications 1 à 4, dans lequel le conseil à l'utilisateur comprend un conseil selon lequel une orientation vers un médecin doit être recherchée, la mémoire (102) stockant une ou plusieurs pathologies de déclenchement pour recommander l'orientation, l'une ou les plusieurs pathologies de déclenchement comprenant un ou plusieurs symptômes ou une combinaison de symptômes.

6. Appareil (100) selon l'une quelconque des revendications 1 à 5, l'appareil (100) étant configuré pour recevoir une entrée sonore au niveau de l'appareil (100) en provenance d'un utilisateur, et l'appareil (100) étant configuré pour utiliser un modèle acoustique pour associer l'entrée sonore aux informations sur un ou plusieurs sons stockés dans la mémoire, de manière à associer l'entrée sonore reçue à un symptôme de la pluralité de symptômes médicaux.

7. Appareil (100) selon l'une quelconque des revendications 1 à 6, l'appareil (100) étant configuré pour recevoir une image entrée sur l'appareil par un utilisateur, et l'appareil étant configuré pour utiliser un moteur de reconnaissance d'image pour associer l'image reçue à des informations sur une ou plusieurs images stockées dans la mémoire, de manière à associer l'image reçue à un symptôme de la pluralité de symptômes médicaux.

8. Appareil (100) selon l'une quelconque des revendications 1 à 7, dans lequel les informations sur un ou plusieurs sons comprennent un premier son associé à une toux grasse et un second son associé à une toux sèche.

9. Programme informatique comprenant des instructions exécutables par ordinateur qui, lorsqu'elles sont exécutées sur un ou plusieurs processeurs, exécutent :
le stockage, dans une mémoire (102) d'un appareil, d'informations sur une pluralité de symptômes médicaux, et le stockage en outre dans la mémoire d'associations entre un ou plusieurs des symptômes médicaux et des informations sur une ou plusieurs pathologies médicales ;
le stockage d'informations sur au moins des première et seconde invites d'entrée à afficher sur une interface utilisateur de l'appareil, de manière à susciter un ou plusieurs symptômes médicaux chez l'utilisateur, un contenu de la seconde invite d'entrée étant déterminé en fonction d'une réponse reçue à la première invite d'entrée ;
la réception d'une entrée d'un utilisateur (101) via une interface utilisateur de l'appareil, l'entrée comprenant une indication d'un ou de plusieurs symptômes médicaux de l'utilisateur ;
le traitement, à l'aide d'un processeur (104), de l'indication reçue d'un ou de plusieurs symptômes médicaux de l'utilisateur, pour déterminer une pathologie médicale associée à l'indication d'un ou de plusieurs symptômes médicaux ; et
la génération d'une sortie à l'utilisateur sur un écran (106) de l'appareil, la sortie comprenant un conseil à l'utilisateur sur la manière de réagir à la pathologie médicale ; **caractérisé en ce que** le procédé comprend le stockage d'informations sur un ou plusieurs sons dans la mémoire (102), chaque son étant associé à un symptôme de la pluralité de symptômes médicaux, dans lequel les informations sur un ou plusieurs sons comprennent des informations sur un ou plusieurs sons de toux ;
le fait de jouer un ou plusieurs des sons stockés dans la mémoire (102) afin d'aider un utilisateur à indiquer un ou plusieurs symptômes ;
le stockage d'une pluralité d'algorithmes (140, 142), chacun des algorithmes étant associé à une pathologie médicale correspondante, la première invite d'entrée étant comprise dans un premier algorithme (140) associé à une première pathologie médicale, la seconde invite d'entrée étant comprise dans un second algorithme (142) associé à une seconde pathologie médicale, et le passage du premier algorithme au second algorithme en réponse à la détermination que le second algorithme est plus applicable aux symptômes de l'utilisateur que le premier algorithme.
